# EUROPEAN PATENT SPECIFICATION

(11) **EP 1 289 573 B1**
(45) Date of publication and mention of the grant of the patent: **10.06.2009**
(21) Application number: 01900300.3
(22) Date of filing: 02.01.2001
(51) Int. Cl.: A61K 9/12, A61M 11/00, A61M 15/00, A61P 5/48

(54) **A MEDICINAL AEROSOL ANTIDIABETICS FORMULATION**
MEDIZINISCHE AEROSOLFORMULIERUNG VON ANTIDIABETIKA
FORMULATION MÉDICINALE AÉROSOL COMPRENANT DES ANTIDIABÉTIQUES

(30) Priority: 25.01.2000 US 177922 P; 31.10.2000 US 702939
(43) Date of publication of application: 12.03.2003
(73) Proprietor: Aeropharm Technology Incorporated, Edison, NJ 08837 (US)
(72) Inventor: ADJEI, Akwete, L., Bridgewater, NJ 08807 (US); CUTIE, Anthony, J., Bridgewater, NJ 08807 (US); SUN, John, Z., Edison, NJ 08817 (US); SEXTON, Frederick, A., Fair Haven, NJ 07704 (US)
(74) Representative: Boeters, Hans Dietrich
(86) International application number: PCT/US2001/000028
(87) International publication number: WO 2001/054741

(56) References cited:
- EP-A- 0 997 151
- WO-A-94/16756
- WO-A-96/19198
- WO-A-98/22169
- US-A- 5 225 183
- US-A- 5 686 411
- KLONOFF D C: "INHALED INSULIN" DIABETES TECHNOLOGY AND THERAPEUTICS, MARY ANN LIEBERT, LARCHMONT, NY, US, vol. 1, no. 3, 1999, pages 307-313, XP008005851 ISSN: 1520-9156
- HIROTA M ET AL: "GLUCAGON IN THE METABOLIC AND NUTRITIONAL MANAGEMENT AFTER TOTAL PANCREATECTOMY A CASE REPORT" JAPANESE JOURNAL OF SURGERY, vol. 19, no. 5, 1989, pages 586-592, XP009007908 ISSN: 0047-1909
- EVANS A J ET AL: "RECENT DEVELOPMENTS AND EMERGING THERAPIES FOR TYPE 2 DIABETES MELLITUS" DRUG DEVELOPMENT RESEARCH, NEW YORK, NY, US, vol. 2, no. 2, 1999, pages 75-94, XP000997750 ISSN: 0272-4391

## Description

### BACKGROUND OF THE INVENTION

### Field of the Invention

This invention relates to a medicinal aerosol formulation, and more particularly, to a medicinal aerosol formulation comprising a β-cell or α-cell hypoglycemic.

### Description of the Related Art

Delivery of drugs to the lung by way of inhalation is an important means of treating a variety of conditions, including such common local conditions as cystic fibrosis, pneumonia, bronchial asthma and chronic obstructive pulmonary disease and some systemic conditions, including hormone replacement, pain management, immune deficiency, erythropoiesis, diabetes, etc. Steroids, β2 agonists, anti-cholinergic agents, proteins and polypeptides are among the drugs that are administered to the lung for such purposes. Such drugs are commonly administered to the lung in the form of an aerosol of particles of respirable size (less than about 10 µm in diameter). The aerosol formulation can be presented as a liquid or a dry powder. In order to assure proper particle size in a liquid aerosol, particles can be prepared in respirable size and then incorporated into a colloidial dispersion either containing a propellant as a metered dose inhaler (MDI) or air, such as in the case of a dry powder inhaler (DPI). Alternatively, formulations can be prepared in solution form in order to avoid the concern for proper particle size in the formulation. Solution formulations must nevertheless be dispensed in a manner that produces particles or droplets of respirable size.

For MDI application, once prepared an aerosol formulation is filled into an aerosol canister equipped with a metered dose valve. In the hands of the patient the formulation is dispensed via an actuator adapted to direct the dose from the valve to the patient.

WO 98/22169 discloses a device which includes a means for aerosolizing a formulation comprised of insulin, which formulation may be a dry powder formulation, a formulation containing a low boiling point propellant, but is preferably a liquid formulation containing no propellant.

David C. Klonoff in Diabetes Technology and Therapeutics, Volume 1, No. 3, 1999, pages 307 to 313 describes in a review article the state of the art of the administration of insulin by means of inhalation.

What is needed and desired is a stable aerosol formulation for the treatment of diabetes and conditions related thereto.

### SUMMARY OF THE INVENTION

It has surprisingly been found that a novel and stable medicinal aerosol formulation of a β-cell or α-cell hypoglycemic medicament can be obtained without the use of a surfactant, such as sorbitan trioleate. A suitable β-cell hypoglycemic medicament is one selected from the group consisting of an amylin and insulin; however, other medicament agents possessing antidiabetic activity, including the α-cell hypoglycemic glucagon, acetohexamide, chlorpropamide, tolazamide, tolbutamide, and glipizide, as well as any mixture of any two or three of the foregoing β-cell hypoglycemic medicaments may be generally included.

### DETAILED DESCRIPTION OF THE INVENTION

This application makes reference to U.S. Application Serial No. 09/209,228 filed December 10, 1998.

This invention involves a stable aerosol formulation according to claim 1 suitable for delivery which comprises (a) a β-cell hypoglycemic medicament, and (b) a suitable fluid carrier.

A suitable β-cell hypoglycemic medicament is one selected from either an amylin or insulin and any of their derivatives. A suitable synthetic, antidiabetic agent is one selected from an acetohexamide, chlorpropamide, tolazemide, tolbutamide, glipizide, glyburide, glucophage, phentolamine, etc., and a mixture of any two or three of the foregoing medicaments.

The term "insulin" shall be interpreted to encompass natural extracted human insulin, recombinantly produced human insulin, insulin extracted from bovine and/or porcine sources, recombinantly produced porcine and bovine insulin and mixtures of any of these insulin products. The term is intended to encompass the polypeptide normally used in the treatment of diabetics in a substantially purified form but encompasses the use of the term in its commercially available pharmaceutical form, which includes additional excipients. The insulin is preferably recombinantly produced and may be dehydrated (completely dried) or in solution.

The terms "insulin analog," "monomeric insulin" and the like are used interchangeably herein and are intended to encompass any form of "insulin" as defined above wherein one or more of the amino acids within the polypeptide chain has been replaced with an alternative amino acid and/or wherein one or more of the amino acids has been deleted or wherein one or more additional amino acids has been added to the polypeptide chain or amino acid sequences which act as insulin in decreasing blood glucose levels. In general, the "insulin analogs" of the present invention include "insulin lispro analogs," as disclosed in U.S. Pat. No. 5,547,929, insulin analogs including LysPro insulin and humalog insulin, and other "super insulin analogs", wherein the ability of the insulin analog to affect serum glucose levels is substantially enhanced as compared with conventional insulin as well as hepatoselective insulin analogs which are more active in the liver than in adipose tissue. Preferred analogs are monomeric insulin analogs, which are insulin-like compounds used for the same general purpose as insulin such as insulin lispro i.e., compounds which are administered to reduce blood glucose levels.

An "amylin" includes natural human amylin, bovine, porcine, rat, rabbit amylin, as well as synthetic, semi-synthetic or recombinant amylin or amylin analogs including pramlintide and other amylin agonists as disclosed in U.S. Patent No. 5,686,411, and U.S. Patent No. 5,854,215.

For purposes of the formulations of this invention, which are intended for inhalation into the lungs, the medicament is preferably micronized whereby a therapeutically effective amount or fraction (e.g. ninety percent or more) of the medicament is particulate. Typically, the particles have a diameter of less than about 10 microns, and preferably less than about 5 microns, in order that the particles can be inhaled into the respiratory tract and/or lungs.

The particulate medicament or drug is present in the inventive formulations in a therapeutically effective amount, that is, an amount such that the drug can be administered as a dispersion or an aerosol, such as topically, or via oral or nasal inhalation, and cause its desired therapeutic effect, typically preferred with one dose, or through several doses. The particulate β-cell hypoglycemic medicament is administered as an aerosol from a conventional valve, e.g., a metered dose valve, through an aerosol adapter also known as an actuator.

The term "amount" as used herein refers to quantity or to concentration as appropriate to the context. The amount of the β-cell hypoglycemic medicament or mixture of medicaments that constitutes a therapeutically effective amount varies according to factors such as the potency of the particular β-cell hypoglycemic medicament or medicaments used, the route of administration of the formulation, and the mechanical system used to administer the formulation. A therapeutically effective amount of a particular drug or drugs can be selected by those of ordinary skill in the art with due consideration of such factors. Generally a therapeutically effective amount will be from about 0.001 parts by weight to about 5 parts by weight based on 100 parts by weight of the fluid carrier e.g. propellant.

A suitable fluid carrier is selected. A suitable fluid carrier includes air, a hydrocarbon, such as n-butane, propane, isopentane, etc. or a propellant. A suitable propellant is any fluorocarbon, e.g. a 1-6 hydrogen containing flurocarbon such as CHF₂CHF₂, CF₃CH₂F, CH₂F₂CH₃ and CF₃CHFCF₃, a perfluorocarbon, e.g. a 1-4 carbon perfluorocarbon, such as CF₃CF₃, CF₃CF₂CF₃; or any mixture of the foregoing, having a sufficient vapor pressure to render them effective as propellants. Some typical suitable propellants include conventional chlorofluorocarbon (CFC) propellants such as propellant 11,12 and 114 or a mixture of any of the foregoing propellants. Non-CFC propellants such as 1, 1, 1,2-tetrafluoroethane (Propellant 134a),1,1,1,2,3,3,3-heptafluoropropane (Propellant 227) or mixtures thereof are preferred. The propellant is preferably present in an amount sufficient to propel a plurality of the selected doses of the drug from an aerosol canisters.

A suitable stabilizer is selected. A suitable stabilizer is a "water addition". As used herein a "water addition" is an amount of water which (1) is added, either initially with other components of the aerosol formulation, e.g. medicament and fluid carrier, or after the other components, e.g. medicament, fluid carrier, are combined and processed, (2) is in addition to the water which is always present and which develops during processing and/or storage of the aerosol formulation, i.e. "developed" or "nascent" formulation water, and (3) is present in an amount which further stabilizes a medicinal aerosol formulation having nascent formulation water.

An aerosol formulation preferably comprises the water addition in an amount effective to more effectively stabilize the formulation relative to an identical formulation not containing the water addition, i.e. containing only nascent formulation water, such that the drug does not settle, cream or flocculate after agitation so quickly as to prevent reproducible dosing of the drug. Reproducible dosing can be achieved if the formulation retains a substantially uniform drug concentration for about fifteen seconds to about five minutes after agitation.

The particular amount of the water addition that constitutes an effective amount is dependent upon the particular fluid carrier, e.g. propellant, and on the particular drug or drugs used in the formulation. It is therefore not practical to enumerate specific effective amounts for use with specific formulations of the invention, but such amounts can readily be determined by those skilled in the art with due consideration of the factors set forth above. Generally, however, the water addition must be present in a formulation in an amount in excess of the concentration of the nascent formulation water. Such concentration of nascent formulation water typically ranges up to 300 parts by weight per one million parts by weight of the total weight of the aerosol formulation. Accordingly, the water addition in excess of this nascent water concentration typically ranges from about 10 parts by weight to 5000 parts by weight per one million parts by weight of the total aerosol formulation weight. Most preferred is that the concentration of the water addition in excess of this nascent water concentration is from 500 parts by weight to 5000 parts by weight per one million parts by weight of the total weight of the medicinal aerosol formulation.

It is to be emphasized that this is an amount which exceeds the amount of nascent or developed formulation water. It is also to be stressed that preferably this amount of water addition can be added and initially combined with the other components of the formulation, e.g. an amylin, glucogan and fluid carrier, e.g. 1,1,1,2-tetrahydrofluoroehtane. However, the water addition can be added to the resultant formulation after these other components have been processed, e.g. prior to or subsequent to storage.

It has surprisingly been found that the formulation of the invention is stable without the necessity of employing a cosolvent, such as ethanol, or surfactants. However, further components, such as conventional lubricants or surfactants, cosolvents, ethanol, etc., can also be present in an aerosol formulation of the invention in suitable amounts readily determined by those skilled in the art. In this regard, reference is made to U.S. Patent No. 5,225,183. Typically, a co-solvent such as ethanol is added in an amount ranging from 0.5 to 10% by weight of the total weight of the formulation.

A most preferred formulation comprises the medicament, the fluid carrier, the ethanol cosolvent and the water addition, for example, an amylin, 1,1,1,2-tetrafluoroethane, ethanol and the water addition.

Generally the formulations of the invention can be prepared by combining (i) the β-cell hypoglycemic drug or drugs in an amount sufficient to provide a plurality of therapeutically effective doses; (ii) the fluid, e.g. propellant, in an amount sufficient to propel a plurality of doses, e.g. from an aerosol canister; (iii) optionally, the water addition in an amount effective to further stabilize each of the formulations; and (iv) any further optional components e.g. ethanol as a cosolvent; and dispersing the components. The components can be dispersed using a conventional mixer or homogenizer, by shaking, or by ultrasonic energy as well as by the use of a bead mill or a microfluidizer. Bulk formulations can be transferred to smaller individual aerosol vials by using valve to valve transfer methods, pressure filling or by using conventional cold-fill methods. It is not required that a component used in a suspension aerosol formulation be soluble in the fluid carrier, e.g. propellant. Those that are not sufficiently soluble can be coated onto the drug particles in an appropriate amount and the coated particles can then be incorporated in a formulation as described above.

Aerosol canisters equipped with conventional valves, preferably metered dose valves, can be used to deliver the formulations of the invention. It has been found, however, that selection of appropriate valve assemblies for use with aerosol formulations is dependent upon the particular component and other adjuvants used (if any), on the fluid, e.g. propellant, and on the particular drug being used. Conventional neoprene and buna valve rubbers used in metered dose valves for delivering conventional CFC formulations often have less than optimal valve delivery characteristics and ease of operation when used with formulations containing HFC-134a or HFC-227. Therefore certain formulations of the invention are preferably dispensed via a valve assembly wherein the diaphragm is made of a nitrile rubber such as DB-218 (American Gasket and Rubber, Schiller Park, Ill.) or an EPDM rubber such as Vistalon^{™} (Exxon), Royalene^{™} (UniRoyal), bunaEP (Bayer). Also suitable are diaphragms fashioned by extrusion, injection molding or compression molding from a thermoplastic elastomeric material such as FLEXOMER^{™} GERS 1085 NT polyolefm (Union Carbide).

Conventional aerosol canisters, coated or uncoated, anodized or unanodized, e.g., those of aluminum, glass, stainless steel, polybutyl or polyethylene terephthalate, and coated canisters or cans with epon, epoxy, etc., can be used to contain a formulation of the invention.

The formulation of the invention can be delivered to the respiratory tract and/or lung by oral inhalation in order to treat diabetes and a diabetes related condition susceptible of treatment by inhalation. The formulations of the invention can also be delivered by nasal inhalation in order to treat, e.g., diabetes (systemic), or they can be delivered via oral (e.g., buccal) administration in order to treat, e.g., diabetes and a diabetes related condition.

## Claims

1. A medicinal aerosol formulation, which comprises;
(a) a therapeutically effective amount of a β-cell hypoglycemic medicament;
(b) a fluid carrier; and
(c) a stabilizer comprising a water addition in an amount which (i) is in addition of nascent formulation water and (ii) is present in an amount to stabilize the formulation to prevent settling, creaming or flocculation for a time sufficient to allow reproducible dosing of the drug after agitation of the formulation.

2. A formulation as defined in claim 1 wherein said *β*-cell hypoglycemic medicament is selected from the group consisting of an amylin, an insulin and a mixture of any of the foregoing.

3. A formulation as defined in claim 1 wherein said fluid carrier is selected from the group of propellents consisting of 1,1,1,2-tetrafluoroethane, 1,1,1,2,3,3,3,-haptafluoropropane and a mixture thereof.

4. A formulation as defined in claim 1 wherein said fluid carrier is a hydrocarbon selected from the group consisting of n-butane, propane, isopentance and a mixture of any of the foregoing.

5. A formulation as defined in claim 1 which further includes a cosolvent.

6. A formulation as defined in claim 5 wherein said cosolvent is ethanol.

7. A method of preparing a medicinal aerosol formulation according to claim 1, which comprises:
(a) combining
(i) said medicament in an amount sufficient to provide a plurality of therapeutically effective doses, with
(ii) said fluid carrier in an amount sufficient to propel a plurality of said therapeutically effective doses from an aerosol canister; and
(b) dispersing components (i) and (ii).

8. A method as defined in claim 7 **characterised in that** step (a) further comprises combining (iii) a stabilizer in an effective amount and wherein step (b) comprises dispersing components (i) and (ii) with said stabilizer.

9. A method as defined in claim 8 **characterised in that** step (a) further comprises combining (iv) a cosolvent and wherein step (b) comprises dispersing components (i), (ii) and (iii) with said cosolvent.

10. Use of a formulation according to claim 1 in the manufacture of a medicament for the treatment or prevention of diabetes or a diabetes related condition capable of treatment by oral or nasal inhalation, said treatment or prevention comprising administering said formulation by oral or nasal inhalation.

11. A formulation according to claim 1 in an aerosol canister equipped with a metered dose valve.

12. A metered dose inhaler containing a medicinal aerosol formulation, said formulation comprising;
(a) a therapeutically effective amount of a *β*-cell hypoglycemic medicament;
(b) a fluid carrier; and
(c) a stabilizer comprising a water addition in an amount which (i) is in addition of nascent formulation water and (ii) is present in an amount to stabilize the formulation to prevent settling, creaming or flocculation for a time sufficient to allow reproducible dosing of the drug after agitation of the formulation.

13. The metered dose inhaler as defined in claim 12 wherein said stabilizer is present in an amount of about 10 parts by weight to about 5000 parts by weight based on one million parts by total weight of the medicinal aerosol formulation.

14. The metered dose inhaler as defined in claim 12 wherein said *β*-cell hypoglycemic agent is selected from the group consisting of an amylin, an insulin and a mixture of the foregoing.

15. The metered dose inhaler as defined in claim 12 which further comprises a synthetic antidiabetic agent.

16. The metered dose inhaler as defined in claim 15 wherein said sythetic antidiabetic agent is selected from the group consisting of acetohexamide, chlorpropamide, tolazemide, tolbutamide, glipizide, glyburide, glucophage and a mixture of any of the foregoing agents.

17. The metered dose inhaler as defined in claim 12 wherein said fluid carrier is selected from the group of propellants consisting of 1,1,1,2-tetrafluoroethane, 1,1,1,2,3,3,3,-haptafluoropropane and a mixture thereof.

18. The metered dose inhaler as defined in claim 12 wherein said fliud carrier is a hydrocarbon selected from the group consisting of n-butane, propane, isopentane and a mixture of any of the foregoing.

19. The metered dose inhaler as defined in claim 12 which further includes a cosolvent.

20. The metered dose inhaler as defined in claim 12 wherein said cosolvent is ethanol.

## Patentansprüche

1. Medizinische Aerosol-Formulierung, welche umfasst:
(a) eine therapeutisch wirksame Menge eines in Bezug auf β-Zellen hypoglykämischen Medikaments;
(b) einen flüssigen Träger;
(c) einen Stabilisator, der eine Zugabe von Wasser in einer Menge umfasst, die
(i) zusätzlich zu dem Wasser vorliegt, welches in der entstehenden Formulierung vorhanden ist, und
(ii) in einer Menge vorliegt, um die Formulierung zu stabilisieren, so dass ein Absetzen, ein Cremigwerden oder ein Ausflocken über einen Zeitraum verhindert wird, welcher ausreicht, um eine reproduzierbare Dosierung des Arzneimittels nach dem Schütteln der Formulierung zu ermöglichen.

2. Formulierung wie in Anspruch 1 definiert, wobei das in Bezug auf β-Zellen hypoglykämische Medikament aus der Gruppe ausgewählt wird, die aus einem Amylin, einem Insulin und einer Mischung von beliebigen der vorstehend genannten Substanzen besteht.

3. Formulierung wie in Anspruch 1 definiert, wobei der flüssige Träger aus der Gruppe von Treibmitteln ausgewählt wird, die aus 1,1,1,2-Tetraflourethan, 1,1,1,2,3,3,3-Heptafluorpropan und einer Mischung derselben besteht.

4. Formulierung wie in Anspruch 1 definiert, wobei der flüssige Träger ein Kohlenwasserstoff ist, der aus der Gruppe ausgewählt wird, die aus n-Butan, Propan, iso-Pentan und einer Mischung von beliebigen der vorstehend genannten Substanzen besteht.

5. Formulierung wie in Anspruch 1 definiert, welche darüber hinaus ein Co-Lösungsmittel umfasst.

6. Formulierung wie in Anspruch 5 definiert, wobei das Co-Lösungsmittel Ethanol ist.

7. Verfahren zur Herstellung einer medizinischen Aerosol-Formulierung nach Anspruch 1, welches Verfahren umfasst:
(a) das Mischen
(i) des Medikaments in einer Menge, die ausreicht, um eine Mehrzahl von therapeutisch wirksamen Dosen bereitzustellen, mit
(ii) dem flüssigen Träger in einer Menge, die ausreicht, um eine Mehrzahl von therapeutisch wirksamen Dosen aus einem Aerosol-Behälter herauszutreiben; und
(b) das Dispergieren der Bestandteile (i) und (ii).

8. Verfahren wie in Anspruch 7 definiert, **dadurch gekennzeichnet, dass** der Schritt (a) darüber hinaus das Mischen (iii) eines Stabilisators in einer wirksamen Menge umfasst, und wobei der Schritt (b) das Dispergieren der Bestandteile (i) und (ii) mit dem Stabilisator umfasst.

9. Verfahren wie in Anspruch 8 definiert, **dadurch gekennzeichnet, dass** der Schritt (a) darüber hinaus das Mischen (iv) eines Co-Lösungsmittels umfasst, und wobei der Schritt (b) das Dispergieren der Bestandteile (i), (ii) und (iii) mit dem Co-Lösungsmittel umfasst.

10. Verwendung einer Formulierung nach Anspruch 1 zur Herstellung eines Medikaments für die Behandlung oder Vorbeugung von Diabetes oder einer Krankheit, die mit Diabetes in Beziehung steht, welche einer Behandlung durch orale oder nasale Inhalation zugänglich sind, wobei die Behandlung oder Vorbeugung die Verabreichung der Formulierung durch orale oder nasale Inhalation umfasst.

11. Formulierung nach Anspruch 1 in einem Aerosol-Behälter, der mit einem regulierbaren Dosierventil ausgestattet ist.

12. Regulierbarer Dosierinhalator, der eine medizinische Aerosol-Formulierung enthält, wobei die Formulierung umfasst:
(a) eine therapeutisch wirksame Menge eines in Bezug auf β-Zellen hypoglykämischen Medikaments;
(b) einen flüssigen Träger;
(c) einen Stabilisator, der eine Zugabe von Wasser in einer Menge umfasst, die
(i) zusätzlich zu dem Wasser vorliegt, welches in der entstehenden Formulierung vorhanden ist, und
(ii) in einer Menge vorliegt, um die Formulierung zu stabilisieren, so dass ein Absetzen, ein Cremigwerden oder ein Ausflocken über einen Zeitraum verhindert wird, welcher ausreicht, um eine reproduzierbare Dosierung des Arzneimittels nach dem Schütteln der Formulierung zu ermöglichen.

13. Regulierbarer Dosierinhalator wie in Anspruch 12 definiert, wobei der Stabilisator in einer Menge von etwa 10 Gewichtsteilen bis etwa 5.000 Gewichtsteilen vorliegt, bezogen auf eine Million Gewichtsteile der medizinischen Aerosol-Formulierung insgesamt.

14. Regulierbarer Dosierinhalator wie in Anspruch 12 definiert, wobei das in Bezug auf β-Zellen hypoglykämische Mittel aus der Gruppe ausgewählt wird, die aus einem Amylin, einem Insulin und einer Mischung von beliebigen der vorstehend genannten Substanzen besteht.

15. Regulierbarer Dosierinhalator wie in Anspruch 12 definiert, der darüber hinaus ein synthetisches antidiabetisches Mittel umfasst.

16. Regulierbarer Dosierinhalator wie in Anspruch 15 definiert, wobei das synthetische antidiabetische Mittel aus der Gruppe ausgewählt wird, die aus Acetohexamide, Chlorpropamide, Tolazemide, Tolbutamide, Glipizide, Glyburide, Glucophage und einer Mischung von beliebigen der vorstehend genannten Substanzen besteht.

17. Regulierbarer Dosierinhalator wie in Anspruch 12 definiert, wobei der flüssige Träger aus der Gruppe von Treibmitteln ausgewählt wird, die aus 1,1,1,2-Tetraflourethan, 1,1,1,2,3,3,3-Heptafluorpropan und einer Mischung derselben besteht.

18. Regulierbarer Dosierinhalator wie in Anspruch 12 definiert, wobei der flüssige Träger ein Kohlenwasserstoff ist, der aus der Gruppe ausgewählt wird, die aus n-Butan, Propan, iso-Pentan und einer Mischung von beliebigen der vorstehend genannten Substanzen besteht.

19. Regulierbarer Dosierinhalator wie in Anspruch 12 definiert, der darüber hinaus ein Co-Lösungsmittel umfasst.

20. Regulierbarer Dosierinhalator wie in Anspruch 12 definiert, wobei das Co-Lösungsmittel Ethanol ist.

## Revendications

1. Formulation médicinale en aérosol, qui comprend :
(a) une quantité efficace, du point de vue thérapeutique, d'un médicament hypoglycémiant agissant sur les cellules β ;
(b) un véhicule fluide ; et
(c) un stabilisant comprenant une addition d'eau en une quantité qui (i) vient en plus de l'eau de formulation naissante et (ii) est présente en une quantité permettant de stabiliser la formulation pour empêcher une décantation, un crémage ou une floculation pendant un temps suffisant pour permettre le dosage reproductible du médicament après agitation de la formulation.

2. Formulation selon la revendication 1, dans laquelle ledit médicament hypoglycémiant agissant sur les cellules β est choisi dans le groupe constitué par l'amyline, une insuline, et un mélange de n'importe lesquelles des précédentes.

3. Formulation selon la revendication 1, dans laquelle ledit véhicule fluide est choisi dans le groupe de propulseur constitué par le 1,1,1,2-tétrafluoroéthane, le 1,1,1,2,3,3,3-heptafluoropropane, et leurs mélanges.

4. Formulation selon la revendication 1, dans laquelle ledit véhicule fluide est un hydrocarbure choisi dans le groupe constitué par le n-butane, le propane, l'isopentane et un mélange de n'importe lesquels des précédents.

5. Formulation selon la revendication 1, qui contient en outre un co-solvant.

6. Formulation selon la revendication 5, dans laquelle ledit co-solvant est l'éthanol.

7. Procédé de préparation d'une formulation médicinale en aérosol selon la revendication 1, qui comprend :
(a) la combinaison
(i) dudit médicament en une quantité suffisante pour constituer une pluralité de doses efficaces du point de vue thérapeutique, avec
(ii) ledit véhicule fluide en une quantité suffisante pour propulser une pluralité desdites doses efficaces du point de vue thérapeutique depuis un bidon aérosol ; et
(b) la dispersion des composants (i) et (ii).

8. Procédé selon la revendication 7, **caractérisé en ce que** l'étape (a) comprend en outre la combinaison de (iii) un stabilisant en une quantité efficace, et dans lequel l'étape (b) comprend la dispersion des composants (i) et (ii) avec ledit stabilisant.

9. Procédé selon la revendication 8, **caractérisé en ce que** l'étape (a) comprend en outre la combinaison de (iv) un co-solvant, et dans lequel l'étape (b) comprend la dispersion des composants (i), (ii) et (iii) avec ledit co-solvant.

10. Utilisation d'une formulation selon la revendication 1 dans la fabrication d'un médicament destiné au traitement ou à la prévention du diabète ou d'un état lié au diabète, capable d'un traitement par inhalation orale ou nasale, ledit traitement ou prévention comprenant l'administration de ladite formulation par inhalation orale ou nasale.

11. Formulation selon la revendication 1 dans un bidon aérosol équipé d'une valve doseuse.

12. Inhalateur doseur contenant une formulation médicinale en aérosol, ladite formulation comprenant :
(a) une quantité efficace, du point de vue thérapeutique, d'un médicament hypoglycémiant agissant sur les cellules β ;
(b) un véhicule fluide ; et
(c) un stabilisant comprenant une addition d'eau en une quantité qui (i) vient en plus de l'eau de formulation naissante et (ii) est présente en une quantité permettant de stabiliser la formulation pour empêcher une décantation, un crémage ou une floculation pendant un temps suffisant pour permettre le dosage reproductible du médicament après agitation de la formulation.

13. Inhalateur doseur selon la revendication 12, dans lequel ledit stabilisant est présent en une quantité d'environ 10 parties en poids à environ 5000 parties en poids pour un total de 1 million de parties en poids de la formulation médicinale en aérosol.

14. Inhalateur doseur selon la revendication 12, dans lequel ledit agent hypoglycémiant agissant sur les cellules β est choisi dans le groupe constitué par une amyline, une insuline et un mélange des précédentes.

15. Inhalateur doseur selon la revendication 12, qui comprend en outre un agent antidiabétique synthétique.

16. Inhalateur doseur selon la revendication 15, dans lequel ledit agent antidiabétique synthétique est choisi dans le groupe constitué par l'acétohexamide, le chlorpropamide, le tolazémide, le tolbutamide, le glipizide, le glyburide, le glucophage et un mélange de n'importe lesquels des agents précédents.

17. Inhalateur doseur selon la revendication 12, dans lequel ledit véhicule fluide est choisi dans le groupe de propulseurs constitué par le 1,1,1,2-tétrafluoroéthane, le 1,1,1,2,3,3,3-heptafluoropropane et un de leurs mélanges.

18. Inhalateur doseur selon la revendication 12, dans lequel ledit véhicule fluide est un hydrocarbure choisi dans le groupe constitué par le n-butane, le propane, l'isopentane et un mélange de n'importe lesquels des précédents.

19. Inhalateur doseur selon la revendication 12, qui contient en outre un co-solvant.

20. Inhalateur doseur selon la revendication 12, dans lequel ledit co-solvant est l'éthanol.
